# EUROPEAN PATENT APPLICATION

(11) **EP 3 025 746 A1**
(43) Date of publication of application: **01.06.2016**
(21) Application number: 14830272.2
(22) Date of filing: 18.07.2014
(51) Int. Cl.: A61M 5/315

(54) **GASKET, AND PLUNGER, SYRINGE, AND PREFILLED SYRINGE WHICH USE SAME**

(30) Priority: 22.07.2013 JP 2013151242
(71) Applicant: Nipro Corporation, Osaka-shi, Osaka 531-8510 (JP)
(72) Inventor: KUBO, Tomohiko, Osaka-shi, Osaka 531-8510 (JP)
(74) Representative: Bertsch, Florian Oliver
(86) International application number: PCT/JP2014/069137
(87) International publication number: WO 2015/012206

(57) **Abstract**

[Issue to be addressed] A structure that prevents gouging and/or tearing caused in producing a gasket when a screw is formed and a die is released.

[Means for solution] A gasket provided with a female screw hole formed as a recess from a base end toward a leading end, said female screw hole including a shaft receiving hole and a screw groove formed in said shaft receiving hole, said shaft receiving hole having a diameter gradually reduced from the base end. The gasket is also applicable for a plunger, a syringe, and a prefilled syringe, and the gasket may include a looseness prevention mechanism.

## Description

### TECHNICAL FIELD

The present invention relates to a gasket and a plunger using the same, a syringe, and a prefilled syringe. More specifically, it relates to a gasket including a structure preventing gouging of a screw hole.

### BACKGROUND ART

When a syringe is normally produced, a plunger rod is previously press-fit into a gasket to provide an assembly of the gasket and the plunger rod, and the assembly is then inserted into a barrel. In contrast, when a drug solution-filled syringe, a so-called prefilled syringe, is produced, normally, a barrel is filled with a drug solution and a gasket is knocked into the barrel and thereafter a plunger rod is coupled with the gasket. In other words, a method is adopted that couples a plunger rod in a subsequent step with a syringe having a gasket knocked therein.

This is done because a gasket with a plunger rod previously attached thereto makes the knocking step difficult. The gasket of the prefilled syringe and the plunger rod are coupled generally in such a manner, as described in Patent Document (PTD) 1, that the female screw of the gasket, formed of an elastic rubber material such as elastomer, and the male screw of the plunger rod, formed of plastic such as polypropylene for example, are engaged with each other (see PTD 1).

This is because a gasket inserted in a barrel is radially less expandable, and pressing and thus fitting a plunger rod to the gasket, as done for a normal syringe, causes a pressure, which may cause the drug solution to leak from the gasket and/or a leading end nozzle.

One method of producing a gasket is as follows: A rubber sheet is placed and pressed between upper and lower dies allowing a plurality of gaskets to be molded. The upper die as referred to herein corresponds to the gasket's leading end and the lower die as referred to herein corresponds to the gasket's base end (including a female screw hole). When pressed, the rubber sheet will be a sheet having a plurality of gaskets formed therein. After the rubber sheet has been pressed and the upper die is then removed, the sheet having the plurality of gaskets formed therein remains on the lower die, and the sheet is removed from the lower die and has the gaskets cut off the sheet to thus provide a large number of gaskets.

### CITATION LIST

### PATENT DOCUMENT

PTD 1: Japanese Patent Laying-Open No. 2000-140103

### SUMMARY OF INVENTION

### TECHNICAL PROBLEM

However, when a gasket has a conventional, straightly shaped screw hole, as seen in PTD 1, and a sheet having a plurality of such gaskets formed therein is removed from a lower die, the gasket(s) may have its/their female screw(s) gouged and/or torn. In that case, there are a possibility that the screw(s) may be impaired in strength and a possibility that dust results and thus contaminates the drug solution.

While the gasket can be formed in a method other than the method of forming it from a sheet, as indicated above by way of example, and it may alternatively be formed in a variety of methods, such as a method introducing a source material of rubber into a die having a space formed therein in the form of the gasket, a method punching the gasket out of a rubber sheet at a stroke, and the like, the methods are all basically the same in that a source material of rubber is molded by using a die and accordingly, the step of removing the gasket's female screw portion from the die is essential somewhere in the process, and the problem of gouging and/or tearing of the female screw is thus unavoidable.

Thus, an issue to be addressed by the present invention is to provide a structure that prevents a gasket from being gouged and/or torn in producing the gasket when a screw is formed in a die and the die is then released.

### SOLUTION TO PROBLEM

Accordingly, to address the above issue, the present inventor has diligently repeated researches, and, as a result, arrived at the present invention. More specifically, the present invention is:
(1) a gasket provided with a female screw hole formed as a recess from a base end toward a leading end, said female screw hole including a shaft receiving hole and a screw groove formed in said shaft receiving hole, said shaft receiving hole having a diameter gradually reduced from the base end. This configuration can reduce the screw's deformation otherwise caused when the rubber sheet is removed from the die as the rubber sheet is pulled, so that gouging and/or tearing otherwise caused when the die is released can be avoided and there is no possibility that the screw is impaired in strength due to gouging and/or tearing and/or that dust is generated by gouging and/or tearing and thus contaminates the drug solution.

Furthermore, the present invention (2) may be utilized as a plunger formed of a plunger rod having a leading end with a male screw engaging with said female screw hole of the gasket of the item (1) above, and said gasket, and (3) may comprise in the plunger of the item (2) above a looseness prevention mechanism provided at a base end of said female screw hole and a base end of said male screw.

Furthermore,(4) it is used as a prefilled syringe comprising the plunger of the items (2) or (3) above and a barrel receiving the gasket of said plunger therein slidably.

### ADVANTAGEOUS EFFECT OF INVENTION

The gouging prevention structure according to the present invention can prevent a gasket's screw hole from being gouged and/or torn in producing the gasket when the die is removed from the gasket.

### BRIEF DESCRIPTION OF DRAWINGS

Fig. 1 is a longitudinal cross section of one exemplary embodiment of a gasket of the present invention.
Fig. 2 is a bottom view of the gasket of Fig. 1.
Fig. 3 is a front view of one exemplary embodiment of a plunger rod of the present invention.
Fig. 4 is a cross section of a screw portion in Fig. 3.
Fig. 5 shows a gasket and a plunger rod combined together, i.e., a plunger.
Fig. 6 shows a prefilled syringe with a plunger and a barrel combined together.

### BEST MODE FOR CARRYING OUT THE INVENTION

Hereinafter reference will be made to the drawings to describe a gasket and a plunger rod according to the present invention. However, the present invention is not limited to any exemplary embodiment shown in these drawings.

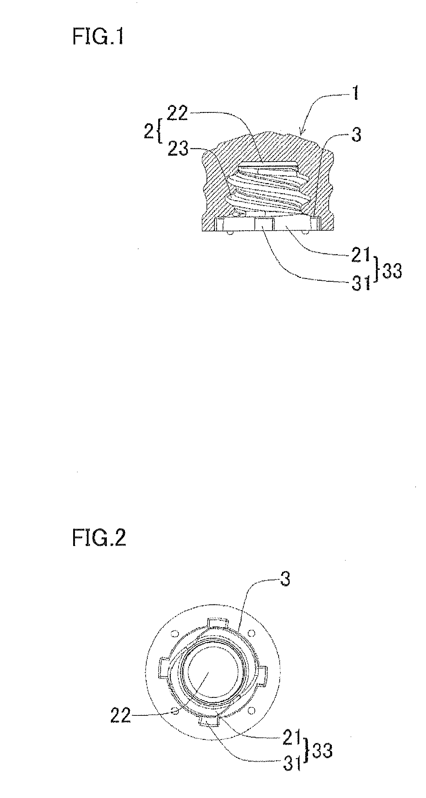

Initially, one exemplary embodiment of the present invention will be described with reference to Fig. 1 and Fig. 2. A gasket body 1 has a female screw hole 2 formed therein from a base end toward a leading end in the form of a recess, and female screw hole 2 is formed of a shaft receiving hole 22 and a screw groove 23 formed in shaft receiving hole 22. Shaft receiving hole 22 is a generally truncated cone having a diameter gradually reduced in a direction from the base end toward the leading end and the depth of screw groove 23 from the shaft receiving hole is uniform. Note that, at the leading end of the shaft receiving hole, the screw groove is processed to be gradually reduced in depth, as is done for a conventional screw. Furthermore, at the base end of gasket 1, an engaged portion 33 is provided that is a portion of a looseness prevention mechanism 3, which will be described hereinafter. Engaged portion 33 is formed of a columnar space 21 contiguous to the base end of female screw hole 2, and four recesses 31 formed to be integral with a periphery of columnar space 21 and each spaced from an adjacent recess by an interval of 90 degrees and recess 31 is dented toward the leading end.

While gasket 1 can be formed of butyl rubber, silicone rubber, thermoplastic elastomer, silicone elastomer or the like, it may be formed of any material that has been proved as having been used as a medical tool and has conventionally been used as a material of a gasket.

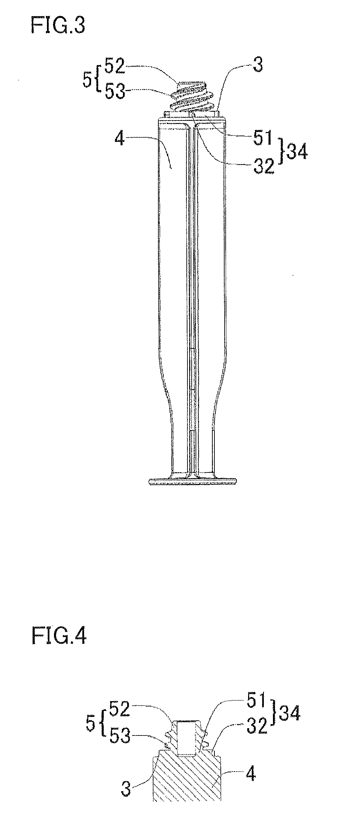

Gasket 1 can be coupled with a plunger rod 4 and they can thus be used as a plunger 6. The plunger rod will now be described with reference to Figs. 3 and 4. Plunger rod 4 has a male screw 5 engaging with female screw 2 of gasket 1 and male screw 5 is formed of a shaft 52 and a screw thread 53 formed on shaft 52. Shaft 52 has a diameter gradually reduced as seen in the direction from the base end to the leading end, and screw thread 53 has a height equal to the depth of screw groove 23. At the base end of male screw 5, an engaging portion 34 is formed that is a portion of looseness prevention mechanism 3, which will be described hereinafter. Engaging portion 34 is provided to be contiguous to the base end of male screw 5 and is formed of a pedestal 51 generally identical in shape to columnar space 21 and four projections 32 integrated with a periphery of pedestal 51 and each spaced from an adjacent projection by an interval of 90 degrees, and generally identical in shape to four recesses 31, with projections 32 projecting toward the leading end of male screw 5.

While plunger rod 4 can be formed of polyethylene, polypropylene or a similar plastic material, it may be formed of any material that has been proved as having been used as a medical tool and has conventionally been used as a material used to form a plunger rod.

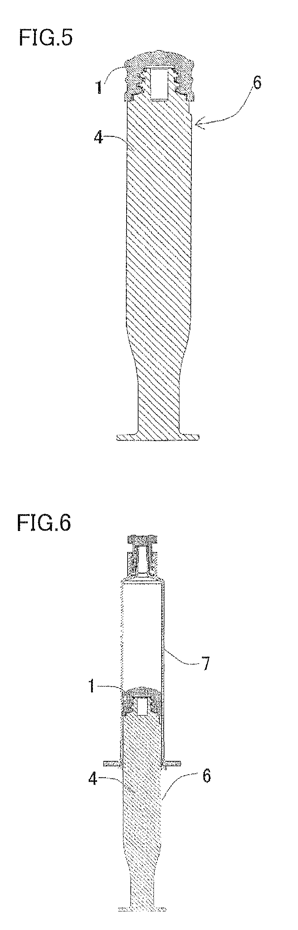

Fig. 5 shows gasket 1 and plunger rod 4 combined together, or plunger 6. Looseness prevention mechanism 3 provided to said gasket 1 and said plunger rod 4 is configured such that projection 32 provided at the base end of male screw 5 of plunger rod 4 engages with recess 31 provided at the base end of gasket 1. This configuration prevents rotation in a direction permitting gasket 1 and plunger rod 4 to be unscrewed and thus prevents loosening. Note that when the looseness prevention mechanism is provided, at least one recess 31 suffices, and the looseness prevention mechanism may have any shape other than shown in shown in Fig. 2, that can prevent rotation in a direction permitting gasket 1 and plunger rod 4 to be unscrewed and thus loosened. Note that while the present embodiment is provided with a looseness prevention mechanism by way of example, the looseness prevention mechanism is not an essential component and there is no problem if it is dispensed with.

According to the illustrated embodiment, the depth of screw groove 23 of female screw 2 and the height of screw thread 53 of male screw 5 are both fixed and furthermore, the depth of screw groove 23 and the height of screw thread 53 are equal, and the screw's strength is thus sufficiently ensured while the shaft receiving hole and the shaft are shaped to be gradually reduced in diameter. Note that the depth of screw groove 23 and the height of screw thread 53 may not be fixed.

Furthermore, as shown in Fig. 6, gasket 1 of the present invention can be coupled with plunger rod 4 and they can thus be presented as plunger 6, and gasket 1 of plunger 6 can be slidably accommodated in the interior of barrel 7 so that they can be used as a syringe or a prefilled syringe.

### INDUSTRIAL APPLICABILITY

As described above, the gasket of the present invention can reduce gouging and/or tearing of a screw hole, and can thus be suitably used for medical care.

### REFERENCE SIGNS LIST

1: gasket
2: female screw
21: columnar space
22: shaft receiving hole
23: screw groove
3: looseness prevention mechanism
31: recess
32: projection
33: engaged portion
34: engaging portion
4: plunger rod
5: male screw
51: pedestal
52: shaft
53: screw thread
6: plunger
7: barrel

## Claims

1. A gasket provided with a female screw hole formed as a recess from a base end toward a leading end, said female screw hole including a shaft receiving hole and a screw groove formed in said shaft receiving hole, said shaft receiving hole having a diameter gradually reduced from the base end.

2. A plunger formed of: a plunger rod having a leading end with a male screw engaging with said female screw hole of the gasket according to claim 1; and said gasket.

3. A plunger that is the plunger according to claim 2, comprising a looseness prevention mechanism provided at a base end of said female screw hole and a base end of said male screw.

4. A syringe comprising the plunger according to claim 2 or 3 and a barrel receiving a gasket of said plunger therein slidably.

5. A prefilled syringe comprising the plunger according to claim 2 or 3 and a barrel receiving a gasket of said plunger therein slidably.
